# EUROPÄISCHE PATENTSCHRIFT

(11) **EP 0 857 478 B1**
(45) Veröffentlichungstag und Bekanntmachung des Hinweises auf die Patenterteilung: **18.06.2003**
(21) Anmeldenummer: 98101961.5
(22) Anmeldetag: 05.02.1998
(51) Int. Cl.: A61K 7/13

(54) **Oxidationshaarfärbemittel**
Oxidative hair dye
Composition de teinture d'oxydation des cheveux

(30) Priorität: 08.02.1997 DE 19704850
(43) Veröffentlichungstag der Anmeldung: 12.08.1998
(73) Patentinhaber: KPSS-Kao Professional Salon Services GmbH, 64297 Darmstadt (DE)
(72) Erfinder: Lorenz, Heribert, 64401 Gross-Bieberau (DE); Kufner, Frank, 64297 Darmstadt (DE)

(56) Entgegenhaltungen:
- EP-A- 0 400 330
- EP-A- 0 467 026
- EP-A- 0 634 163
- EP-A- 0 722 710
- EP-A- 0 728 465
- EP-A- 0 819 423
- WO-A-97/11674
- US-A- 3 834 866

## Beschreibung

Die vorliegende Erfindung betrifft ein Haarfärbemittel auf Basis eines mit Peroxid reagierenden Oxidationsfarbstoff-systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen ist insofern nicht völlig problemfrei, als sie bei extrem empfindlichen Personen in speziellen Fällen zu Hautsensibilisierungen führen können (bei sogenannten "Para-Allergikern").

Es wurde bereits versucht, dieses Problem durch Verwendung alternativer Entwicklersubstanzen zu lösen. Dies ist in beschränktem Umfang möglich durch den Einsatz von Tetraaminopyrimidin oder 2-(2,5-Diaminophenyl)ethanol (vgl. EP-A 7537 und EP-B 400 330); jedoch müssen dann erhebliche Abstriche in der Färbeintensität und den Variationsmöglichkeiten der verschiedenen Farbtöne hingenommen werden.

Eine weitgehend optimale Lösung des Problems, nämlich die Abwesenheit von Hautsensibilisierung einerseits und eine große Variationsbreite der Erzielung möglicher Farbnuancen andererseits, wird durch den in der EP-A 615 743 beschriebenen Einsatz von 2-(2'-Hydroxyethylamino)-5-aminotoluol bzw. dessen wasserlöslichen Salzen und die aus der EP-B 467 026 bekannten Triaminohydroxypyrimidine, insbesondere 2,5,6-Triamino-4-hydroxypyrimidin, 2,4,5-Triamino-6-hydroxypyrimidin, 4,5,6-Triamino-2-hydroxypyrimidin bzw. deren salze, insbesondere die Sulfate, als Entwicklersubstanzen in Haarfärbemitteln erreicht.

Selbst dadurch bleiben jedoch noch farbtechnische Wünsche offen.

Die Erfindung geht daher von der Aufgabenstellung aus, ein Haarfärbemittel zu schaffen, das 2-(2'-Hydroxyethylamino)-5-aminotoluol, 2-(2,5-Diaminophenyl)ethanol, 1-Amino-4-bis(2'hydroxyethyl)aminobenzol, und/oder mindestens ein Triaminohydroxypyrimidin bzw. deren wasserlösliche Salze als Entwicklersubstanz enthält und zur Herstellung einer großen Anzahl von Farbtönen geeignet ist.

Diese Aufgabe wird dadurch gelöst, daß ein solches Haarfärbemittel ein mit Peroxid reagierendes Oxidationsfarbstoff-System enthält, das aus einer Kombination aus mindestens einer Entwicklersubstanz ausgewählt aus der Gruppe 2-(2'-Hydroxyethylamino)-5-aminotoluol, 2-(2,5-Diaminophenyl)ethanol, 1-Amino-4-bis-(2'-hydroxyethyl)aminobenzol, und/oder mindestens einem Triaminohydroxypyrimidin bzw. deren wasserlöslichen Salzen und 5-Amino-2-methoxyphenol besteht.

Bei Anwendung dieser Zusammensetzungen auf Basis einer üblichen Grundlage werden nach der Oxidation mit Peroxid sehr ausdrucksvolle, intensive, dauerhafte Haarfärbungen erhalten, die durch Zusatz entsprechender Kupplersubstanzen hoch zu anderen Farbnuancen variiert werden können.

Solche bevorzugten Kupplersubstanzen sind insbesondere Resorcin, 2-Methylresorcin, 4-Chlorresorcin, 2-Aminophenol, 3-Aminophenol, 1-Methyl-2-hydroxy-4-aminobenzol, 2-Amino-3-hydroxypyridin, 2-Amino-4-β-hydroxyethylaminoanisol bzw. dessen wasserlösliche Salze und α-Naphthol.

Damit soll jedoch der Zusatz weiterer Kupplersubstanzen keineswegs ausgeschlossen sein.

Die Verwendung von 5-Amino-2-methoxyphenol in Haarfärbemitteln ist im Stand der Technik bereits erwähnt worden; durch diese eher vagen Angaben läßt sich jedoch keinerlei Hinweis auf die mit den erfindungsgemäßen Kombinationen erzielbaren Effekte entnehmen.

Auch die zusätzliche Mitverwendung weiterer, an sich bekannter Entwicklersubstanzen ist möglich. Neben den bereits eingangs genannten sind hierbei insbesondere noch 4-Aminophenol, 5-Aminosalicylsäure und/oder 1,2,4-Triaminobenzol zu erwähnen.

Die Gesamtkonzentration der Entwicklersubstanzen liegt zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.

Das Gewichtsverhältnis von 2-(2'-Hydroxyethylamino)-5-aminotoluol, 2-(2,5.Diaminophenyl)ethanol, 1-Amino-4-bis-(2'hydroxyethyl)aminobenzol, bzw. Triaminohydroxypyrimidin zu 5-Amino-2-methoxyphenol liegt dabei zwischen etwa 1 : 8 bis 8 : 1, vorzugsweise etwa 1 : 5 bis 5 : 1, insbesondere 1 : 2 bis 2 : 1.

Die Kupplersubstanz(en) als Reaktionspartner der Entwicklersubstanz(en) liegen in den erfindungsgemäßen Haarfärbemitteln etwa im gleichen Anteil wie die Entwicklersubstanzen vor, d. h., also in Mengen von 0,05 bis 5,0 %, vorzugsweise 0,1 bis 4 %, insbesondere 0,5 bis 3 Gew.-% der Gesamtzusammensetzung (ohne Oxidationsmittel), wobei sich die Angaben jeweils auf den Anteil an freier Base beziehen.

Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.
Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind.

Sie können als Lösungen, Cremes, Gele oder auch in Form von Aerosol-Präparaten vorliegen; geeignete Trägermaterial-Zusammensetzungen sind aus dem Stand der Technik hinreichend bekannt.
Zur Applikation wird das erfindungsgemäße Oxidationsfarbstoffvorprodukt mit einem Oxidationsmittel vermischt. Bevorzugtes Oxidationsmittel ist Wasserstoffperoxid beispielsweise in 2- bis 6-prozentiger Konzentration.

Es können jedoch auch andere Peroxide wie Harnstoffperoxid und Melaminperoxid eingesetzt werden.

Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 9,5 liegen.

Im folgenden werden verschiedene Ausführungsbeispiele zur Erläuterung der Erfindung gegeben.

| | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 8,5 |
| Ammoniumchlorid | 0,5 |
| Panthenol | 0,8 |
| Wasser | @ 100,00 |

Die erfindungsgemäßen Oxidationsfarbstoff-Kombinationen wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung und zwanzigminütiger Einwirkung bei Zimmertemperatur, folgendem Auswaschen und Trocknen.

Es wurden die folgenden Färbungen erzielt:

| | Ergebnis: |
|---|---|
| Beispiel 1: | |
| 0,54 (Gew.-%) 0,54 (Gew.-%) | 4-Hydroxy-2,5,6-triaminopyrimidinsulfat (TRAP) |
| 0,31 | 5-Amino-2-methoxyphenol |

| Färbung: | |
|---|---|
| Kräftiges Rotviolett. | |

| Beispiel 2: | |
|---|---|
| 0,71 (Gew.-%) | 2-(2'-Hydroxyethylamino)-5-aminotoluolsulfat (HAT) |
| 0,31 | 5-Amino-2-methoxyphenol |

| Färbung: | |
|---|---|
| Kräftiges Dunkelviolett. | |

| Beispiel 3: | |
|---|---|
| 0,66 (Gew.-%) | 1-Amino-4-bis-(2'-hydroxyethyl)aminobenzolsulfat (A 50) |
| 0,31 | 5-Amino-2-methoxyphenol |

| Färbung: | |
|---|---|
| Leuchtendes Rotviolettbraun. | |

| Beispiel 4: | |
|---|---|
| 0,56 (Gew.-%) | 2-(2,5-Diaminophenyl)ethanol ethanolsulfat (A 80) |
| 0,31 | 5-Amino-2-methoxyphenol |

| Färbung: | |
|---|---|
| Kräftiges, ausdrucksvolles Violettbraun. | |

| Beispiel 5: | |
|---|---|
| 0,47 (Gew.-%) | HAT |
| 0,21 | 5-Amino-2-methoxyphenol |
| 0,18 | 2-Methylresorcin |

| Färbung: | |
|---|---|
| Kräftiges, glänzendes Violett. | |

| Beispiel 6: | |
|---|---|
| 0,38 (Gew.-%) | A 80 |
| 0,21 | 5-Amino-2-methoxyphenol |
| 0,16 | 3-Aminophenol |

| Färbung: | |
|---|---|
| Glänzendes Violettbraun. | |

| Beispiel 7: | |
|---|---|
| 0,44 (Gew.-%) | A 50 |
| 0,21 | 5-Amino-2-methoxyphenol |
| 0,16 | 3-Aminophenol |

| Färbung: | |
|---|---|
| Kräftiges Blauviolett. | |

| Beispiel 8: | |
|---|---|
| 0,36 (Gew.-%) | TRAP |
| 0,21 | 5-Amino-2-methoxyphenol |
| 0,18 | 2-Methylresorcin |

| Färbung: | |
|---|---|
| Glänzendes Pinkrot. | |

| Beispiel 9: | |
|---|---|
| 0,43 (Gew.-%) | TRAP |
| 0,10 | 5-Amino-2-methoxyphenol |
| 0,20 | 3-Aminophenol |

| Färbung: | |
|---|---|
| Glänzendes Kupfergold. | |

Ersatz des 5-Amino-2-methoxyphenols durch 2-Aminophenol führte in allen Beispielen zu blassen, unansehnlichen Ausfärbungen.

## Patentansprüche

1. Haarfärbemittel auf Basis eines mit Peroxid reagierenden Oxidationsfarbstoff-Systems, enthaltend eine Kombination aus a)mindestens einer Entwicklersubstanz, ausgewählt aus 2-(2'-Hydroxyethylamino)-5-aminotoluol, 2-(2,5-Diaminophenyl)-ethanol, 1-Amino-4-bis-(2'-hydroxyethyl)aminobenzol, und/oder mindestens einem Triaminohydroxypyrimidin bzw. deren wasserlöslichen Salzen und b)5-Amino-2-methoxyphenol.

2. Haarfärbemittel nach Anspruch 1, enthaltend die Bestandteile a) und b) in einem Gewichtsverhältnis zwischen 1 : 5 und 5 : 1.

3. Haarfärbemittel nach einem der Ansprüche 1 oder 2, enthaltend mindestens eine zusätzliche Kupplersubstanz, ausgewählt aus der Gruppe Resorcin, 2-Methylresorcin, 4-Chlorresorcin, 3-Aminophenol, 1-Methyl-2-hydroxy-4-aminobenzol, 2-Amino-3-hydroxypyridin, α-Naphthol, 3-Amino-2-methylamino-6-methoxypyridin und/oder 2-Amino-4-(β-hydroxyethylamino)anisol bzw. dessen wasserlöslichen Salzen.

## Claims

1. Composition for the dyeing of human hair on the basis of an oxidation dyestuff system reacting with peroxide, comprising a combination of
a)at least one developing substance selected from 2-(2'-hydroxyethyl amino)-5-aminotoluene, 2-(2,5-diaminophenyl)-ethanol, 1-amino-4-bis-(2'-hydroxyethyl aminobenzene, and/or at least one triaminohydroxypyrimidine, or water-soluble salts thereof, and
b)5-amino-2-methoxyphenol.

2. Composition for the dyeing of human hair according to claim 1, comprising the components a) and b) in a weight proportion between 1 : 5 and 5 : 1.

3. Composition for the dyeing of human hair according to one of claims 1 or 2, comprising at least one additional coupling agent, selected from the group resorcinol, 2-methyl-resorcinol, 4-chlororesorcinol, 3-aminophenol, 1-methyl-2-hydroxy-4-aminobenzene, 2-amino-3-hydroxypyridine, α-naphthol, 3-amino-2-methyl amino-6-methoxypyridine and/or 2-amino-4-(β-hydroxyethyl amino)anisole, or the water-soluble salts thereof.

## Revendications

1. Colorant pour cheveux à base d'un système de colorant d'oxydation réagissant avec un peroxyde, contenant une combinaison de
a) au moins une substance développeur choisie dans le groupe formé par le 2-(2'-hydroxyéthylamino)-5-aminotoluène, le 2-(2,5-diaminophényl)-éthanol, le 1-amino-4-bis-(2'-hydroxyéthyl) -aminobenzène, et/ou au moins une triaminohydroxypyrimidine ou leurs sels solubles dans l'eau et
b) du 5-amino-2-méthoxyphénol.

2. Colorant pour cheveux selon la revendication 1, contenant les composants a) et b) dans un rapport pondéral compris entre 1 : 5 et 5 : 1.

3. Colorant pour cheveux selon l'une des revendications 1 ou 2, contenant au moins une substance coupleur supplémentaire choisie dans le groupe formé par la résorcine, la 2-méthylrésorcine, la 4-chlororésorcine, le 3-aminophénol, le 1-méthyl-2-hydroxy-4-aminobenzène, la 2-amino-3-hydroxypyridine, le α-naphtol, la 3-amino-2-méthylamino-6-méthoxy-pyridine et/ou le 2-amino-4-(β-hydroxyéthylamino) anisole ou ses sels solubles dans l'eau.
